# EUROPEAN PATENT SPECIFICATION

(11) **EP 1 127 943 B1**
(45) Date of publication and mention of the grant of the patent: **08.10.2008**
(21) Application number: 01200582.3
(22) Date of filing: 19.02.2001
(51) Int. Cl.: C12N 15/74, C10G 32/00

(54) **Means and methods for the expression of homologous and heterologous proteins in strains of Rhodococcus**
Mittel und Verfahren zur Expression von homologen und heterologen Proteinen in Rhodococcus
Moyens et méthodes d'expression de proteines homologues et hétérologues en Rhodococcus

(30) Priority: 24.02.2000 IT MI000332
(43) Date of publication of application: 29.08.2001
(60) Divisional application: 08163664.9
(73) Proprietor: ENI S.p.A., 00144 Rome (IT)
(72) Inventor: Margarit y Ros, Immacolata, 20097 San Donato Milanese (IT); Serbolisca, Luca Paolo, 20133 Milan (IT); De Ferra, Francesca, 20075 Lodi (IT); Rodriguez, Francesco, 20097 San Donato Milanese (IT)
(74) Representative: De Gregori, Antonella

(56) References cited:
- WO-A-98/17787
- DE MOT RENE ET AL: "Structural analysis of the 6 kb cryptic plasmid pFAJ2600 from Rhodococcus erythropolis NI86/21 and construction of Escherichia coli-Rhodococcus shuttle vectors." MICROBIOLOGY (READING), vol. 143, no. 10, 1997, pages 3137-3147, XP001015207 ISSN: 1350-0872
- SERBOLISCA L.; DE FERRA F.; MARGARIT I.: 'Manipulation of the DNA coding for the desulphurizing activity in a new isolate of Arthrobacter sp.' APPL MICROBIOL BIOTECHNOL no. 52, 1999, pages 122 - 126
- DENIS-LAROSE C. ET AL: 'Cahracterization of the basic replicon of Rhodococcus plasmid pSOX and development of a Rhodococcus-Escherichia coli Shuttle vector' APPLIED AND ENVIRONMENTAL MICROBIOLOGY 1998, pages 4363 - 4367

## Description

The present invention relates to a new constitutive promoter of Rhodococcus, an expression vector containing said promoter, microorganisms transformed with the expression vector and their use in the production of proteins.

Bacteria of the Rhodococcus type are of wide interest in the field of the biodegradation and biotransformation of organic compounds (Warhurst and Fewson, 1994, Crit. Rev. Biotechnol., 14:29-73).

Processes are known, for example, which use strains of Rhodococcus for the selective removal of organic sulfur from fossil fuels (U.S. 5,358,870, U.S. 5,132,219, PCT/US92/01868, EP-445896) and for the production of enzymes involved in the production of acrylamides (Kobayashi et al., 1992, Tends Biotechnol., 10:402-408), carboxylic acids, L-aminoacids (W09804733) and enantiomorphs of chiral compounds (U.S. 5,672,504).

The main restricting factor in optimizing biocatalysis processes which use these bacteria is however the lack of suitable genetic instruments.

This term refers to expression vectors in Rhodococcus which:
- are present in cells in multiple copies;
- are steadily maintained inside the cells without costly selective agents (for example antibiotics), which considerably influence the economic convenience of an industrial process; and
- contain a strong promoter, i.e. capable of allowing an effective expression of a gene, or a strong constitutive promoter which does not require the use of inductors and is not susceptible to repressors.

In fact, a limit in the removal of organic sulfur from fossil fuels with strains of Rhodococcus which produce the sox enzymatic complex, is due to the presence, upstream the corresponding genes, of a promoter greatly inhibited by sulfate.

To overcome this drawback, the genes encoding this enzymatic complex were placed in Rhodococcus vectors under the control of constitutive heterologous promoters such as that of the gene for resistance to chloramphenicol of Rhodococcus fascians (Piddington, C.S. et al., 1995, Appl. and Env. Microbiol., 61,2,: 468-475) or of the gene sacB of B.subtilis (Denis-Larose, C. et al, 1998, Appl. and Env. Microbiol., 64,11,:4363-4367) and of the gene for resistance to Kanamycin of E.coli (Serbolisca et al., Appl. Microbiol. Biotechnol. 1999, 52:122-126). The maintenance of the vectors, however, required the presence of a selective agent in the culture medium and, furthermore, the expression of the sox operon under the control of the promoter sac B proved to be very low (Lau. P. et al. 1999 ACS Fuel Chem.:32-33).

Another prior art document discloses cloning and expression vectors for Rhodococcus, wherein the rep genes repA and repB, par A gene and chloramphenicol reistance gene were used (De Mot Rene et al., Microbiology 1997, 143, 10: 3137-3147).

The objective technical problem solved by the present invention is to provide an alternative promoter of Rhodococcus and expression vector to be used for the removal of organic sulfur from fossil fuels. vector.

It has now been found that the disadvantages of the known art described above can be overcome by means of the expression vector of the present invention.

A first objective of the present invention relates to a new constitutive promoter of Rhodococcus capable of directing the expression of a homologous or heterologous gene with a high efficiency and characterized by the sequence SEQ. ID. Nr. 2.

Another objective of the present invention relates to an expression vector in Rhodococcus which comprises said constitutive promoter.

A further objective of the present invention relates to a strain of Rhodococcus transformed with said expression

Yet another objective of the present invention relates to a process for the production of homologous or heterologous proteins in Rhodococcus bacteria transformed with said expression vector.

Additional objectives of the present invention will appear evident from the following description and examples.

### Brief description of the figures

Figure 1: shows the restriction map of the 11 kb plasmid pSM841.
Figure 2: shows the restriction map of the 7.3 kb plasmid pSM843.
Figure 3: shows the restriction map of the E.coli plasmid pSM839.
Figure 4: shows the restriction map of the plasmid pSM846.
Figure 5: shows the restriction map of the plasmid pSM847.

In particular, the expression vector according to the present invention comprises:
(a) the rep genes, ORF81 and trbA which encode proteins involved in replication in Rhodococcus;
(b) a gene called parA whose product is necessary for maintaining the plasmid in the absence of selective pressure and is characterized by the sequence SEQ ID. Nr. 1;
(c) a constitutive promoter of Rhodococcus having the sequence SEQ. ID. Nr. 2;
(d) a multiple cloning site downstream the promoter; and
(e) at least one gene which encodes a genetic marker selected, for example, from genes of the cad operon (SEQ. ID. Nr. 3), which provide resistance to cadmium or genes which encode resistance to an antibiotic.

The expression vector also contains the replication origin in E.coli and can therefore be used as shuttle vector in Rhodococcus and E.coli.

The expression vector, indicated hereunder as pSM846, was obtained by:
(1) construction of the cloning vector pSM843 comprising:
   (a) the rep genes, ORF81 and trbA which encode proteins involved in replication in Rhodococcus;
   (b) the gene parA having the sequence SEQ. ID. Nr. 1; and
   (c) at least one gene which encodes a genetic marker selected from the genes of the cad operon, which confer resistance to cadmium, or the genes which encode resistance to an antibiotic
(2) isolation of a constitutive promoter of Rhodococcus having the sequence SEQ ID No:2 ; and
(3) insertion of said constitutive promoter into the vector pSM843.

The plasmid vector pSM843 was prepared by reducing the dimensions of the 130 kb plasmid of Rhodococcus sp. DS7 containing the sox operon, the genes which confer resistance to cadmium, and those for resistance to arsenic (Margarit et al., 1997 and Serbolisca et al., 1999). This reduction was effected by means of the following strategy:
(a) search for plasmids deriving from the deletion of the 130 kb plasmid following transformation of a recipient strain and isolation of a 22 kb plasmid;
(b) digestion of said plasmid with suitable restriction enzymes, self-ligase and isolation of the 11 kb plasmid pSM841;
(c) characterization of the plasmid obtained in (b) and
(d) construction of the 7.3 kb plasmid pSM843 containing at least one genetic marker and the genes parA, rep and trbA respectively necessary for stability and replication in Rhodococcus.

The stability of the vectors obtained was controlled after each reduction so as to select only those maintained in at least 90% of the cells of the host strain for at least 30-40 generations, in complete medium and in the absence of selective pressure.

Research on constitutive promoters inside the chromosome of a strain of Rhodococcus was effected for the construction of the expression vector, using a new method.

This method is based on the observation that strains of Rhodococcus have the capacity of integrating at random fragments of foreign DNA in their chromosome, without the necessity for a sequence homology higher than 3 bp between the donor DNA and that of the host. The integration effectiveness was estimated at about 10²-10³ colonies per µg of DNA in transformation experiments of cells of Rhodococcus.

The research method consists in:
(i) transforming a strain of Rhodococcus directly with a gene reporter without its promoter or with a multicopy plasmid of E. coli containing said gene and linearized upstream the gene reporter;
(ii) selecting the clones which express said gene, i.e. the clones which have integrated the gene reporter in their chromosome, downstream a promoter sequence;
(iii) digesting the chromosomal DNA of the clones selected with restriction enzymes which cut upstream and downstream the gene;
(iv) amplifying the DNA obtained in step (iii); and
(v) sequencing the promoter upstream the gene reporter.

Gene reporter refers to a fragment of DNA which encodes a product that allows the selection of the clones which express it. Examples of gene reporters useful for this purpose can be selected from those which encode resistance to antibiotics or heavy metals or enzymes such as XylE or the same Sox proteins.

With respect to the techniques currently used, this research method is much more efficient and rapid as it does not require the preparation of genome banks with fragments of chromosomal DNA before the gene reporter. This method can be applied to all micro-organisms which, like Rhodococcus, are capable of integrating at random fragments of foreign DNA in their chromosome without the necessity for a high sequence homology between the donor DNA and that of the host.

With this method, a constitutive promoter was identified of a gene of Rhodococcus having the sequence SEQ. ID Nr. 2 which is included in the scope of the present invention. This promoter was inserted into the plasmid vector psM843, obtaining the expression vector pSM846 which can be used for the production of proteins of interest in Rhodococcus.

The segregational and structural stability of the expression vector pSM846 in strains of Rhodococcus was determined by operating as described in Example 4. The results demonstrated that the vector is maintained in over 90% of the cellular population even after subsequent culture passages without selective agents, thus showing a high segregational stability.

Furthermore, analysis of the plasmids isolated from the transformed strains showed that, even after various generations in liquid culture, this plasmid remains structurally stable in different strains of Rhodococcus with from 4 to 8 copies per cell.

The expression vector of the present invention can be used for the expression of genes which encode proteins of interest such as, for example, enzymes involved in the selective removal of organic sulfur from fossil fuels (SoxA, SoxB, SoxC, SoxD), the production of L-aminoacids (amidase, aspartase), the production of enantiomorphs of chiral compounds (epoxide hydrolase, ketoesteroreductase), the production of carboxylic acids (nitrilase), etc.

The expression vector can be used in various species of Rhodococcus and in phylogenetically similar bacteria such as Gordona and Nocardia.

The capacity of the promoter of directing the constitutive expression of the gene put under its control was verified by positioning downstream said promoter, the sox operon isolated according to what is described by Serbolisca, L., de Ferra, F., Margarit, I., Appl. Microbiol. Biotechnol., 1999, 52:122-126.

The results obtained demonstrated that this promoter allows an effective and constitutive expression of the SoxA, SoxB and SoxC proteins in the presence of inorganic sulfur in the culture medium.

The strains containing the vectors pSM843, pSM846 and pSM847 were deposited at the Centraalbureau Voor Schimmelcultures as Rhodococcus SMV 112, SMV 113 and SMV 114, where they received the respective numbers CBS 102445, 102446 and 102447.

The following were used in the experiments described hereunder:
- the 130 Kb plasmid containing the sox operon which enencode resistance to cadmium and arsenic. Said plasmid was isolated from the strain Rhodococcus sp. DS7 (Margarit, I. et al., 1997, 9th Proceedings of the International Conference on Coal Science: 1579-1582);
- the plasmid pSM789, incapable of replicating itself in Rhodococcus, obtained by inserting into the plasmid of E.coli pUC18, the 4549 bp fragment HindIII-EcorRI which contains the sox operon without promoter isolated from Rhodococcus sp. DS7 (Margarit, I. et al., 1997, 9th Proceedings of the International Conference on Coal Science: 1579-1582).

The Rhodococcus sp. DS7 strain was previously classified as Arthrobacter on the basis of microbiological test (D'Addario 1996 Proceedings of the Symposium AAA Biotechnology. Shejbal, E. and Ferrara: 139-149). Subsequently, a comparison of the DNA 16 S sequence with that of the data banks indicated that the strain belonged to the Rhodococcus genus. The most consistent homologies were found with Rhodococcus erithreus and Rhodococcus erythropolis.
- the Rhodococcus DS-2 strain, obtained from Rhodococcus sp. DS7, incapable of desulfurizing as it does not have the 130 kb plasmid (Margarit, I. et al., 1997, 9th Proceedings of the International Conference on Coal Science: 1579-1582).

The following examples are illustrative but do not limit the scope of the invention itself.

### EXAMPLE 1

### Construction of the plasmid vector pSM843

Electrocompetent cells of Rhodococcus DS-2 (100 µl) (BIORAD Gene Pulser TM, 400 ohm, 25 uF) were transformed with the 130 kb plasmid (100 ng). The recombinant clones were selected on plates of LB-agar containing 0.5 mM of CdCl₂ and subsequently plated on minimum medium (10 g/l of KH₂PO₄ pH 7.4, 2.5 g/l of NH₄Cl, 0.2 g/l of MgCl₂·6H₂O, 0.02 g/l of CaCl₂, 0.01 g/l of FeCl₃, 0.005 g/l of MnCl₂·2H₂O, 0.003 g/l of ZnCl₂, 0.0009 g/l of CuCl₂·2H₂O, agarose 0.8%) containing dibenzothiophene (DBT) as sole sulfur source. One of the cadmium-resistant clones proved to be incapable of desulfurizing DBT. The plasmid DNA was extracted from this clone, which, after being tested on agarose gel at 0.7%, showed dimensions of about 100 kb.

Operating as described above, from the transformation of DS-2 with the 100 kb plasmid, a 35 kb plasmid was obtained which no longer conferred resistance to arsenic, and from this a 22 kb plasmid.

The plasmid stability was checked for the three vectors in order to select only those maintained in the host strain for at least 30-40 generations in the absence of selective pressure.

The 22 kb plasmid (1 µg) was digested with 4 U of the restriction enzyme Sex AI (Boehringer), in 10 µl of the salts supplied by the producer, incubating for 60 minutes at 37°C and 15 minutes at 70°C. The linearized plasmid (2 µl) was self-ligated in 10 µl of buffer, in the presence of 1 U of T4 DNA ligase, for 16 hours at 16°C. 1 µl of the ligase mixture was used to transform DS-2 electrocompetent cells. A 18.7 kb plasmid was isolated from one of the clones containing plasmids with reduced dimensions. This plasmid was digested with the enzyme EcoRI obtaining a 15.8 kb vector which was further reduced by digestion with the enzymes SspI and DraI, which leave truncated ends.

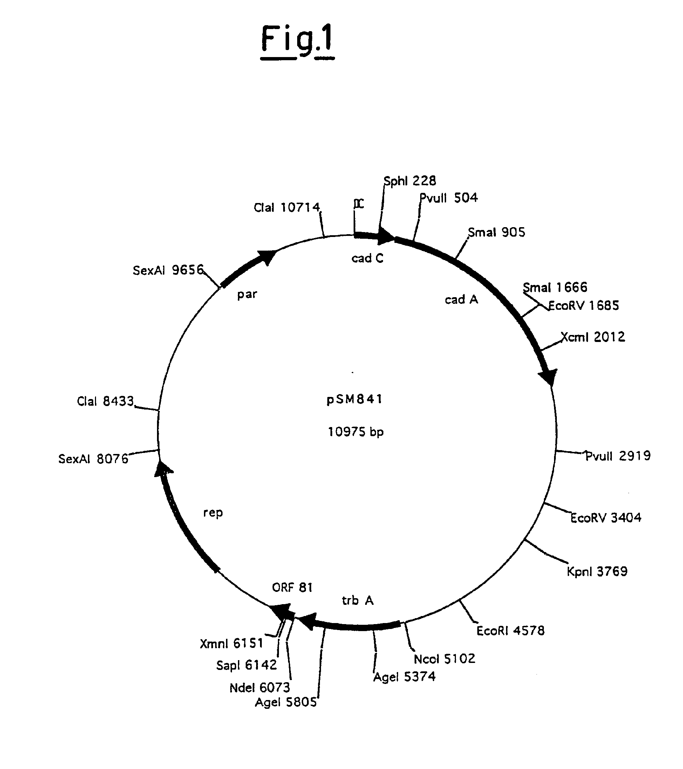

The resulting 11 kb plasmid, called pSM841 (figure 1), was sequenced and showed the presence of:
- the cad operon which confers resistance to cadmium and having the sequence SEQ. ID. Nr. 3;
- rep genes, ORF81 and trbA encoding proteins involved in replication described by Denis-Larose, C. et al., 1998, Appl. and Env. Microbiol., 64, 11: 4363-4367. A 15 bp sequence was identified between these two genes, which, by homology with other sequences, may correspond to the replication origin of the plasmid; and
- a gene parA whose sequence (SEQ ID Nr. 1) shows a partial homology with that of genes involved in the partioning of the plasmids which takes place during the cellular division. 2 repeated sequences each of 24 bp, were identified of the plasmids which takes place during the cellular division. 2 repeated sequences each of 24 bp, were identified upstream this gene.

To confirm the importance of the DNA region containing the parA gene in maintaining pSM841, an 8.8 kb plasmid was constructed without this region. The results demonstrated a reduction of over 50% in the plasmidic stability.

Once the genes indispensable for maintaining the plasmid pSM841 had been identified, a 7.3 kb vector was constructed, containing:
1- a fragment DraI-EcoRI of 2717 bp comprising the cad operon;
2- a fragment SspI-NcoI of 2716 bp comprising the genes trb A and rep A;
3- a fragment EcoRI-NcoI of 1857 bp comprising the gene par A and a multiple cloning site (MCS).

These fragments were isolated from the plasmid pSM841 by means of amplification and then ligated in the presence of T4 DNA ligase at 16°C for a night.

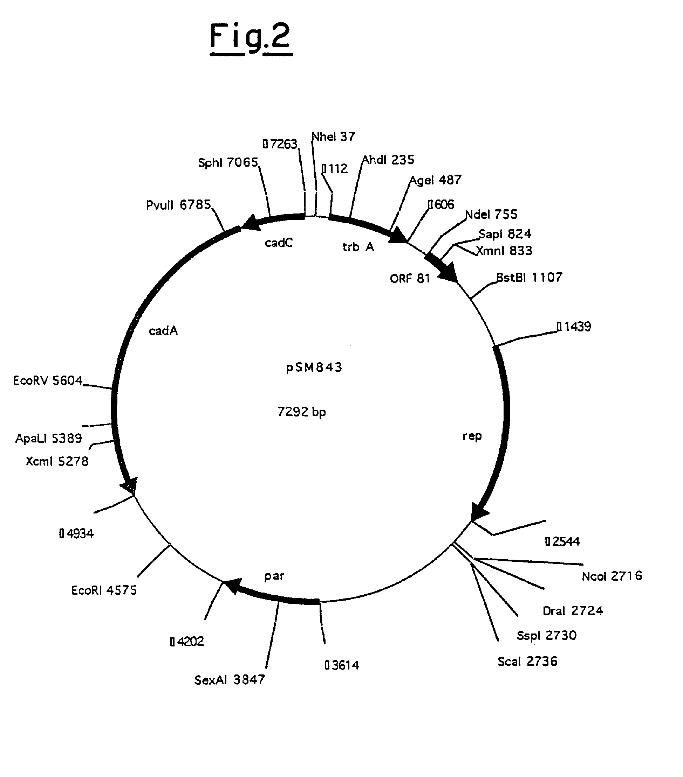

The resulting plasmid, called pSM843, has the restriction map indicated in figure 2.

### EXAMPLE 2

### Isolation of the constitutive promoter

The plasmid pSM789 (5 µg) was digested with 40 Units (U) of the restriction enzyme HindIII (Boehringer) in 50 µl of buffer supplied by the manufacturer.

After incubation at 37°C for 60 minutes the denaturation/extraction of the proteins was effected by adding a volume of phenol-chloroform (1:1) and a volume of chloroform:isoamyl alcohol (23:1). The DNA was subsequently precipitated by adding 5 µl of a 3 M solution of Na-acetate and 300 µl of ethanol to the aqueous phase and then resuspended in 10 µl of H₂O. In this way, an open linear plasmid was obtained exactly upstream the RBS region of the first gene of the sox operon (sox A).

The linearized DNA (0.2 µg) was used to transform 100 µl of cells of Rhodococcus sp. DS-2 and the transformants were then selected on minimum medium plates containing 0.04 g/l of DBT and 1% of C₂H₅OH as sole sources of sulfur and carbon, respectively.

100 clones capable of growing were isolated using DBT. As the plasmid pSM789 was incapable of replicating itself in Rhodococcus, the transformants obtained (about 10² per µg of DNA used) had to contain the sox operon inside their chromosome, downstream a promoter which allowed its expression.

In order to determine whether the integration took place in different points of the chromosome, the chromosomal DNA was extracted from 20 clones (Current Protocols in Molecular Biology - Vol 1) and digested with 20 U of the enzyme NotI, for which there is only one binding sequence electrophoresis on agarose gel 0.8%, visualized by colouring with EtBr 0.5% and transferred onto nylon (Nylon Membranes, positively charged - Boehringer) by means of Southern Blot (Sambrook, J., Fritsch, E.F., Maniatis, T. 1989 Molecular cloning: a laboratory manual 2nd edn. Cold Spring Harbor, NY).

The DNA was then hybridized with a probe whose nucleotidic sequence corresponded to a fragment of 770 bp of the sox C gene, using the non-radioactive method provided by the DIG SYSTEM- kit Boehringer. The hybridization reaction was effected at 68°C.

Of the clones tested a single band was observed, with a different molecular weight for each clone, which indicated a single integration event of the plasmid pSM789 in different points of the chromosome.

In order to verify if these clones were capable of desulfurizing DBT in the presence of inorganic sulfur, they were plated on minimum medium containing both DBT and MgSO₄·7H₂O (0.20 g/l), in parallel with the Rhodococcus sp. DS7 strain. After 12 hours, upon exposing the plate to UV rays at 254 nm, a fluorescent halo was observed for all the recombinant clones, whereas no halo was visible for the native strain. This indicated that the activity of the isolated promoters was not repressed by sulfate. The clone which had the most consistent halo was called SMV110.

### EXAMPLE 3

### Characterization of the promoter contained in SMV110

1 µg of chromosomal DNA of the clone SMV110 was digested with 20 U of the enzyme ClaI, in 10 µl of buffer (Boehringer), at 37°C for 16 hours. After deactivation of the enzymes at 70°C for 15 minutes, 2 µl of the digested DNA was treated with 1 U of the enzyme T4 DNA ligase in 10 µl of buffer (Boehringer), incubating at 16°C for 16 hours.

An aliquot (1 µl) of the ligase mixture was used to transform cells of E.coli XL1-Blue made electrocompetent (Dower, W.J. et al. 1988 Nucleic Acids Research, 16: 6127-6145).

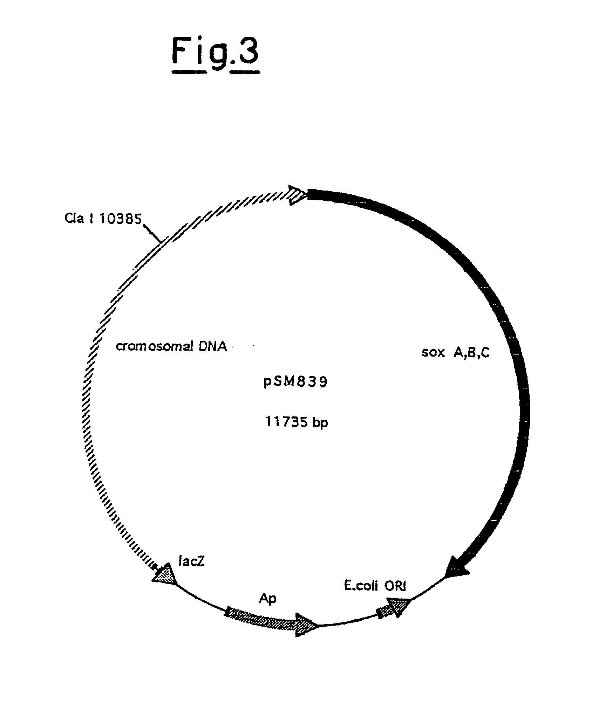

The transformants were selected on plates of agarized LB medium containing 100 µg/ml of Ampicillin. The plasmid DNA extracted from one of the clones thus obtained was analyzed by restriction analysis. The results indicated that the plasmid consisted of a 4.5 kb fragment, deriving from the chromosomal DNA of DS-2, and the plasmid pSM789. The map of this new plasmid, called pSM839, is showed in figure 3.

The 4.5 kb fragment was then amplified with the Polymerase Chain Reaction (PCR) technique, (Leung, D.W., Chen, E., Goeddel, D.V., 1989 Technique- a journal methods in cell and molecular biology, 1, Nr. 1: 11-15), using the following pair of oligonucleotides:
1) 5' CAGTCACGAC GTTGTAAAAC GA 3' (FORWARD)
2) 5' TGCATTTGTC GTTGTTGAGT 3' (REVERSE)

The amplification was carried out in a DNA Thermal Cycler 480 apparatus (Perkin-Elmer Cetus) using 100 µl of a reaction mixture containing: 5 ng of plasmid DNA, 60 pmoles of the two oligonucleotides, 200 µM of dNTPs (dATP, dGTP, dTTP, dCTP) and 1 U of Taq polymerase (Boehringer), in the buffer recommended by the manufacturer. After denaturation for 2 minutes at 94°C, the cyclic program was started, which comprises: 1 minute at 98°C, 1 minute at 60°C and 3 minutes at 72°C for a total of 25 cycles, followed by 8 minutes at 72°C (final extension).

The amplified 4.5 kb fragment was then sequenced by means of a DNA sequencer ABI 373 (Perkin Elmer). The sequence and possible open reading frames were compared with those present in DNA and protein banks using the research motor BLAST supplied by NCBI (Altscul, S.F., Madden, Th, Schaffer, A., Zhang, J., Zhang, Z., Miller, W., Lipman, D., 1997 Nucleic Acids Research 25: 3398-3402).

The results showed that the integration of pSM789 in the chromosomal DNA of the DS-2 strain had taken place inside a gene which encodes a protein homologous to some endoglucanases and amylases of other microorganisms.

It was therefore deduced that in the clone SMV110, the expression of the sox operon was controlled by the promoter expression of the sox operon was controlled by the promoter of said gene. In order to characterize this promoter, the exact transcription starting point of the sox operon was determined by primer extension using the 5' RACE System kit (BRL).

The results indicated that the start of the transcription of the sox operon took place at a distance of 622 bp from 5' of the operon and that the promoter was therefore situated immediately upstream said region.

Analysis of the sequence upstream the transcription starting site revealed the presence of the presumed -10 region.

### EXAMPLE 4

### Construction of the expression vector pSM846

A 1100 bp fragment containing the constitutive promoter was amplified by means of PCR starting from the plasmid pSM839 using the following oligonucleotides:
(i) 
5'GATCGGCCGGG ATCCACGAGT GTT 3' (forward)

BamHI
(ii)

5'ACCCAACACT GA GCTGTTAA CGGCCGGAGC GGCCGATGCA

HindIII HpaI SfiI

TTTTAGGTGA TGCCCGGG 3′ (reverse).

Nsi

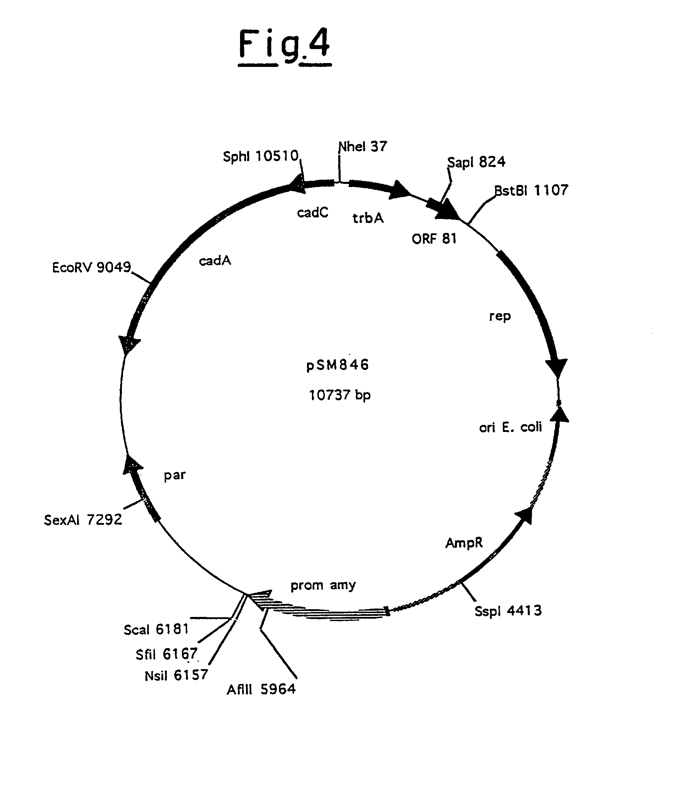

The amplified fragment was digested with the enzymes HindIII and BamHI, eluted from agarose and inserted into the enzymes AflIII-HpaI and ligated to pSM843, previously digested with AflIII and NcoI (which form compatible ends with the former) in the presence of T4 DNA ligase at 16°C for a night. A new vector shuttle of E.coli-Rhodococcus, called pSM846, was thus obtained, containing the promoter followed by an MCS for the constitutive expression of proteins in Rhodococcus. The strain of Rhodococcus DS-2 containing the plasmid pSM846, whose map is showed in figure 4, was called SMV112.

### EXAMPLE 5

In order to verify the stability of the plasmid pSM846 in the transformed strains of Rhodococcus after a prolonged period of culture in the absence of selective pressure, three independent clones of the strain SMV112 were grown at 30°C, 200 rpm for 16 hours in 100 ml flasks, containing 20 ml of LB medium (Bacto Triptone 10 g/l, yeast extract 5 g/l, NaCl 10 g/l).

The three cultures (0.1 ml) were used to inoculate a further 20 ml of the same mediums, and the new cultures were grown at 30°C, 200 rpm for 16 hours. This procedure was repeated a further 3 times, the cellular growth being followed as an increase of the optic density measured at 600 nm (O.D. 600).

At the end of each growth, aliquots of cultures were removed, suitably diluted and then plated on LB agar medium. The plates were incubated at 30°C for 16 hours and the colonies were then counted (CFU/ml). In this way it was determined that there were at least 35 generations at the end of the experiment.

In order to determine the percentage of cells which had maintained the plasmid for at least 35 generations, and were therefore resistant to cadmium, 100 single colonies deriving from the plating by dilution, after the 5^{th} growth of the three cultures, were placed on LB plates containing CdCl₂ at a concentration of 0.5 mM.

It was observed that the plasmid was maintained in over 90% of the cellular population, thus showing a considerable segregational stability even after subsequent passages in culture in the absence of selective pressure.

In order to verify the structural stability of the plasmid pSM846, at the end of the 5^{th} growth passage, the plasmid DNA was extracted from an aliquot of the cultures of 12 clones, using the method described in Serbolisca et al. (1999). The DNA obtained were digested with various restriction enzymes for which there are single or double sites in the plasmid pSM846 and analyzed by means of electrophoresis on agarose gel for 2 hours at 90 V, 90 mA; the gels were coloured with Ethidium Bromide 1 mg/ml. The visualization of the gel on a UV light trans-illuminator showed the expected bands according to the restriction map of the plasmid, demonstrating that pSM846 was structurally stable in Rhodococcus for several generations in the absence of selective pressure.

### EXAMPLE 6

### Determination of the number of copies of pSM846 per cell

The average number of copies of the plasmid pSM846 per cell was estimated by means of quantitative PCR on preparations of total DNA from cells of Rhodococcus DS-2 containing said plasmid. The method is based on the determination of the quantity of DNA obtained by amplification of a fragment of the plasmid. This quantity is directly correlated to the concentration of the plasmid itself, used as a mould in the amplification reaction.

The total DNA was prepared according to the method illustrated in Current Protocols in Molecular Biology - Ausubel et al., Ed. Wiley Interscience Section 2.4.1 with the following modification: to obtain a complete cellular lysis the centrifuged cells from 1.5 ml of culture were resuspended in 567 microliters of TE (TrisHCl 10 mM, EDTA 1 mM, pH 8) containing lysozyme 50 µg/ml and were incubated at 37°C for 20 minutes before adding SDS and proteinase K according to protocol.

After a treatment of 10 minutes with RNasi, the concentration of total DNA was estimated on agarose gel and the samples were diluted to 100 pg of DNA per microliter.

PCR reactions were effected on the DNA thus obtained, using two oligonucleotides (primers) which appear inside the operon for resistance to cadmium and having the following sequence:
- 5' TGGCCCGGCC GGAATTGATG GAC 3' (primer Cd4) and
- 5' GCCGACGGCC GCGATCGTGA TCAG 3'(primer Cd17).

The standardization was effected by means of a second PCR reaction on the portion of chromosomal DNA encoding RNA 16S of Rhodococcus DS7 using oligonucleotides specific for this sequence.

The number of copies of plasmid was estimated on both the strain containing the plasmid pSM846 and on a strain containing a single copy of the genes for resistance to cadmium integrated in the chromosome and on the strain DS7.

The amplification reactions were carried out according to the instructions of the Syber Green kit of Perkin Elmer-AB.

The amplified DNA was marked with the Syber Green fluorescent marker and quantified with a PE Applied Biosystems GeneAmp 5700 instrument. Each sample was triply amplified and the fluorescence values were compared with calibration curves included in each experiment for each pair of primers. The quantity of DNA obtained by amplifying with the primers specific for the plasmid was then corrected for the quantity of chromosomal DNA amplified with the primers specific for the RNA 16S genes.

On comparing the three strains tested it was established that the strain DS7 contains an average of 2-3 copies of the 130 kb plasmid per cell, whereas cells of Rhodococcus DS-2 transformed with pSM846 contain from 4 to 8 copies of plasmid per cell.

### EXAMPLE 7

### Construction of the plasmid pSM847

The objective of the experiment was to obtain a strain containing several copies of sox genes under the control of the constitutive promoter identified.

The fragment of DNA containing the promoter identified in example 3, was amplified starting from the plasmid pSM839 by means of PCR, using the following oligonucleotides:
- 5' ATTCGAGAGT GCATATGCGG AAC 3' (FORWARD)
- 5' CCATTTCTTC CAAGCTTCCG CCG 3' (REVERSE)
which pair with the sequence SEQ. ID. Nr. 2, into which the restriction sites NdeI and HindIII were introduced.

About 500 ng of the DNA obtained from the amplification reaction were digested with 4 U of the enzymes NdeI and HindIII for 60 minutes at 37°C and purified on Nusieve agarose gel at 1.5% (FMC products). Parallelly, 1 µg of the plasmid pSM789 was digested with 4 U of the same enzymes for 60 minutes at 37°C. 50 ng of vector and 25 ng of insert purified from agarose were then ligated in the presence of 1 U of the enzyme T4 ligase. The ligase mixture was subsequently used to transform competent cells of E.coli XL-blue.

500 ng of the plasmid extracted from one of the clones of E.coli containing the desired insert, and 500 ng of the plasmid pSM843 were digested separately with 4 U of the restriction enzyme SspI (New England Biolabs). The reactions were carried out at 37°C for 60 minutes and at 70°C for 10 minutes.

150 ng of the linear plasmid pSM843 were then ligated with 50 ng of the plasmid of E.coli in 10 µl of buffer (Boehringer), in the presence of 1 U of T4 DNA ligase, incubating for 16 hours at 16°C. 1 µl of the ligase mixture was used to transform electrocompetent cells of Rhodococcus DS-2. The cells were plated on minimum medium in the presence of DBT and cultivated at 37°C for 4-5 days.

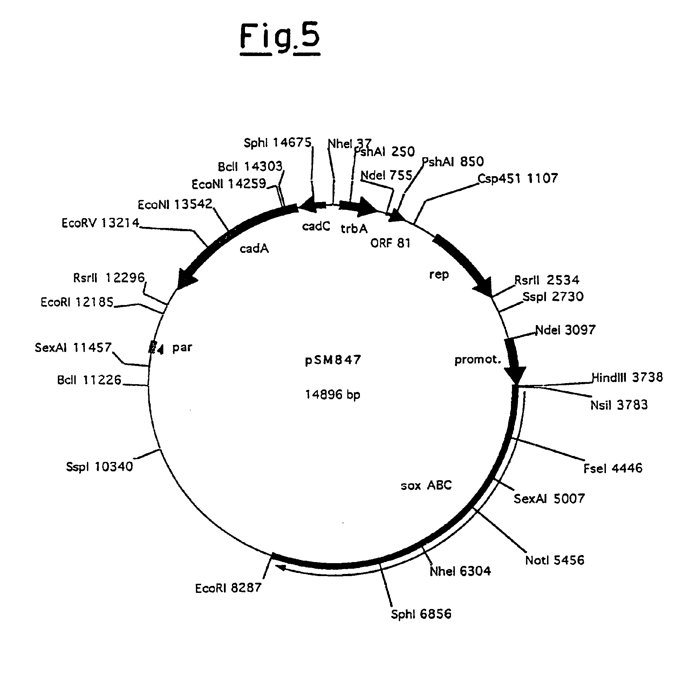

The plasmid DNA was extracted from one of the colonies thus obtained, according to the method described in Serbolisca et al. (1999, Appl. Env. Microbiol. 52:122-126) and subjected to restriction analysis with enzymes for which there are sites in the starting plasmids. The vector thus obtained, containing the sox operon under the control of the constitutive promoter, was called pSM847 (Figure 5) and the strain containing it SMV114.

### EXAMPLE 8

The strain SMV114 was cultivated in parallel with the strain Rhodococcus DS7 in minimum medium plus DBT with and without 0.20 g/l of MgSO₄·7H₂O.

Electrophoresis and Western blot analysis of the soluble proteins extracted from the bacterial cultures showed that:
1. when the strains are grown in DBT without sulfate, SMV114 expresses higher quantities of Sox A, B and C proteins than DS7;
2. in the presence of inorganic sulfate, the strain Rhodococcus DS7 does not express the Sox enzymes, whereas SMV114 expresses the same quantity of enzymes with respect to growth without sulfate.

The desulfurizing activity of the strain SMV114 was measured on resting cells using DBT as substrate and compared with that of the strain Rhodococcus sp. DS7. From a pre-inoculum in minimum medium, two inocula were effected for each strain in 100 ml of the same medium, without and with inorganic sulfur (0.20 g/l of MgSO₄·7H₂O, 80 mM). The growths were effected for 20 hours at 30°C in flasks with breakwater protection. The starting optical density, measured at 660 nm, was OD 0.25 for the cultures in MgSO₄ + DBT and 0.4 for the cultures with DBT alone. The dry weight was triply determined, from 5 ml of centrifuged culture at 5000-6000 rpm for 10 minutes, frozen at -80°C and lyophilized.

For the measurement of the desulfurizing activity, 10 ml of culture were removed and centrifuged at 5000-6000 rpm for 10 minutes, at room temperature. The cells were resuspended in 9.75 ml of Tris·HCl 20 mM pH 7, introduced into 50 ml flasks and incubated in a stirred bath at 30°C for 5 minutes. After adding 250 µl of DBT 40 mM to the suspension, a first sampling (1 ml) was effected immediately, which was extracted with 2 ml of ethyl acetate, to determine the product background at time zero. Subsequent samples were taken after 1 and 2 hours.

20 µl of the clarified organic phase were analyzed by HPLC on a chromatographic column in C18 Vydac type TP218-5418 inverse phase, with a program comprising a flow of 1 ml/min for 15 minutes in 80% of acetonitrile. The specific activity, expressed as mg of 2HBP produced per hour per gram of dry weight, was calculated from the area corresponding to the product 2-hydroxybiphenyl (2 HBP) and taking in account the dry weight.

The activity values obtained without inorganic sulfur corresponded to 5 +/- 0.6 mg/h.g both for the native strain and for SMV114. When cultivated in the presence of 0.2 g/l of MgSO₄, the Rhodococcus DS7 strain did not express any desulfurizing activity, whereas the SMV114 strain maintained its activity.

### EXAMPLE 9

The possibility of using the plasmid pSM847 in other species of Rhodococcus was also examined. 100 ng of the plasmid were used to transform electrocompetent cells of strains of Rhodococcus erythropolis, Rhodococcus rhodochrous and Rhodococcus opacus. In all cases colonies capable of desulfurizing DBT were obtained, which contained the plasmid having the estimated dimensions. Also in this case, at least 90% of the clones maintained the plasmid for about 40 generations in the absence of selective pressure.

### SEQUENCE LISTING

SEQ ID NO: 1
   SEQUENCE TYPE: Nucleotide
   SEQUENCE LENGTH: 2290 base pairs
   STRANDEDNESS: Single
   TOPOLOGY: Linear 
ATCGATTTTT CCGCCGGCGG CGCCGCCGCA TCCCACGCTG CCGTGAGTCC 50

GTCCAAAGCT CGCAGCATCG ACGGGTGGTA CTTACGCAAC GCTGCCTGGC 100

TGAACCCGGC GTTGAGGATG ATGTGCGCCT TCTGCCAGTT GGGTTCGGAG 150

TTGAAAGCGG TGAAAAGGCC GTCCTCCGCG ACTGCCCGCA GCTTTCGGAT 200

CGGCAGACCC ACCGATTTCG CCCACAATGC CTCGTTGTTG ACCTCGGCGA 250

CCAGCTCCAC CCCGGAGACC ACGGTCAGTC GGTGATTGAT GATCTTGCGC 300

TCGAAGATCG GCCCCAGCCT CGAAGCCATG ACCATTTCCT TCTGGACAGG 350

CTTCACCGAG TCGACGCTGA GCAAGTCCCC CAACACAGGC AGCCGCCAGG 400

ACGGCGACGG AATCGACTCG AACTCAAGGA CTGGTTTGAC CGCGACCTCG 450

CTGGTGGGTG TCTCACTGCT CGGATACGGG CACTTGTCGG AAGACATGGT 500

GGACAGTCTT GCATCAGTTC CGGACTAGAT GCCGACTCGA CGCAAAATGG 550
 
GCGATGGAAC GCAAAGCCGC CATCCAGGAT GGAGCAGCGC GCCAAGAACC 600
ATGCAAACCA GCGCTACTTG CGCAGACAAG CGGAAGTCGA ACGTGTCGTT 650
GTTCCCCCTC ATCTTCACTT CCCCACTGCT CTACGGAACC TTCTGAGCTG 700
GCATTTTGCA TGATTTCCGA GCCCCGGAAC CAGCTATACA GTTTTACGGC 750
AAACCGGGAA ACTGGAAAAC CGTATAGACG GCTACCCGGC AAAACGATAT 800
GCTGTCTATA CGGATAGACG GGAAACCGGA TGCCTGGGAA ACTGGCTAAG 850
TGTGTATACG GCTAACTGGC AAGCCGGGAA GCTGTTTTAC AGGTAACTGT 900
TTTTACAGCT TTCCGGCAAA CCGGGAAACT GTAAAACCGG CAAACTGGGA 950
ACTGTAAAAC CGGCAAACTG TAAACAAGCG AAAGGGCCCT TCTCGATGAT 1000
CATCAGTCTC GTCAACACCA AAGGCGGAAC GGCCAAGACG ACGAGCGCGA 1050
TCTACCTTGC ACTCGCGTTT CATAATCGGG GGAGGAAGGT TGTCGTCCTC 1100
GACTTGGATA AACAGGGTTC AGCAACTGAC TGGGCTGACC GCGCCACAGA 1150
GGCCGGAGAT CCACTCCCGT TCCCAGTGCA TGTCGTGAAC ATGAAACGGC 1200
TGGTGAAGTA CGCCACCGAT GGCGATGACC AGGTAGTAAT CATCGACACC 1250
CCGCCCGGTG ACGGGCAAGT TATCGACGCT GCAATCGGGG CGTCCAACTT 1300
CGTCATAATG CCCACGGCGG CTACAGGACT CGACACCGCC AGGGTCTGGG 1350
AGACGCTGCC CTCGGTGCAG GGCCGCCTTC CCTACGGAAT CCTCATTACC 1400
TCCGCACGTC TCGGAACGAA CCTGCTTGAA GATGCCAAGG CAGCGTTCGA 1450
CAGCAACGAC GCAGCCCGAT TCGACACCGT CATCCCCATG CGCGAGCGCA 1500
TCCGGTCAAC ATTCGGTACT ACCCCCAAGC ACGACGAAGG GTATTCCGAC 1550
GTCGTCGACG AGATCACAGA GGCGCTGACA GCATGAGCAT CCCCAAGGCA 1600
AAGACGACAC CGACGCTCGG GCCCCGCAAG TCCGCGCCAC CGATCGCGAC 1650
GCCCTCAGTG ACGTCCAGCT TCGTCGAACC GGACGCAGAC CGCACGAAAC 1700
TCACCGTCCA GATCGACGCT GAACTGCACC GACGCTTCAA AGCTGCTGTT 1750
GCAGGAAGCG GCAAGAAGAT GCGCGACGTG GTCGAGGAAA TGATCGAGCA 1800
 
GTGGACCGAC GCGAACAGTG GTCGCTCATA ACTCGGTGAC GCTGTTACGC 1850

AAAACCAAAG GGAGCCTCGC CGGTTGCACC CGACGAGGCT CCAGACCGGG 1900

AAACCCCTGG AAGGAAGAAA CCCAGTCATG ACCGACTTTA GCCTTCGGGC 1950

AGCTCGTATT CAGCTAGGCG CATTGCTCGC CGCACTGGCA CTCGCGATCG 2000

TCATTGCGCT CGGTATCACC ACCGGCGCAT TCGTACTTTC CTTCGCCGTG 2050

CAACGCGACC TTGCACGGCA AGCACTGATC CCCGAACACC TGACCTGGAT 2100

CTTCCCGGCG ATTGTCGACA GCGCTATCCT CGGCGCCACG ATCGCCATCG 2150

TCATCATCAG CAAGCTCAAC ATGAACAAGC GCGACAGAGG CTTCTACATC 2200

GCACTCGCCG TCAGCGTTGT CGTGATCAGC ATCCTCGGAA ACGCGTACCA 2250

CGCCTATCAC GCAGCAATCG CCGCGCAGGA GTCGATCGAT 2290
SEQ ID NO: 2
   SEQUENCE TYPE: Nucleotide
   SEQUENCE LENGTH: 1355 base pairs
   STRANDEDNESS: Single
   TOPOLOGY: Linear
   PROPERTIES: Promoter 
ATCGATCGGC CGGGATCGAC GAGTGTTGCC ATTTCACCGA GCACTTCGCC 50
ACCGCGGATC TGACGGCGCC GATCTCGGTG ATGGAGTCCT GGTCGGCGCT 100
TCCACCTGTG GTCTCGAGGT CGACGACGAC AAACGTCGTT TCGCTCAGCG 150
GTGTGTCCAG CTCGTCGAAG CTCAGTTGCC GCCCGACGTC GAACTTCTCC 200
CCGTCGGAGA ACGGGGACGG AGACGGAAAT TCTCGGGCCG AGGCGGGACT 250
CACACCGCAG ACAGTAGAAG GACCCCCCGA CACGAATTCG AGAGTGCATA 300
CGCGGAACCC GGCTCACTCC GACTTCCATT GGGAAAGCAA CGGATTGACC 350
CCCGACAAAA GTACGAGAGA CGAGGATCAC ACTTGCGGTG GGAACTCGCG 400
AATGCGGACT CCTACCTACT TACCGGTAGG GCGATTCGTG AGGGTGTTGC 450
 
TACGGCGATC AGTCGCCGAA ACCGCCTGAT TGATCGAAAC GGGTTCTAGT 500

TTCAAGTAAT CGGCGTTGAA CTGCATGGAA GATCGGATTG TCGGGTTTGG 550

TGACCCTTGC CAGTGGGTAG CAAGCGCAGG TTTGCGGGCC AGGTGAGCCT 600

CGCCGAACAG ACCTGTTGTT ATCTCTTCGT GACCCGTCCG GCGTATCGCG 650

GGCGCGGCAG CATGATTTCC GGCGAATCGG GCGCGATTGT CACGAAGCGG 700

TCGACATCGT CGCGATCATT TCGTAACCTT CCTGAGACCT AACGAAGTCT 750

TGCCGGACCA AACAGGACCG GCATCGTCAG GAACCGCCTA ATCGGGATCT 800

CCGCGAGGAA CCCGAACCGG GAACCCAAGT TTCCACTGGG GTGAATCCCG 850

CCTGGGGTGC AGCACGCATG ATCGCTGCGG ACCACGCGGG TAGGGCTGAT 900

CTTCCCAGCC CGAACCCGTC AGCTAACTCG GTCGGCGGAT GAATGGAAGA 950

AATGGAGCAC CCCTTAAGTG GCGTCACAGA GTTTCAAGCG TTCAGCGCAG 1000

TTGGCAGTAG CCGGCGCGCT CGCAGTCGGA GCATTTGCTG CAACCGCTGC 1050

ACCTGCCTCG GCAGACCCCA TCACCATCCC CGGCGTCGGC ACCTTCGAGG 1100

TTCCGGGCGC TTCTATTCCT CAGTTGCCGG TCATCCCGGG CATCACCGAC 1150

ATCGCACCGG CCGCTCCGGC CGCTCCCATC TCCAGTGTTG GTGAGCAGGC 1200

AGTTCGCGCC GCAGAGAGCA AGCTGGGCTC CCCGTACGTG TACGGCGCAT 1250

CGGGCCCGGA CGCATTCGAC TGCTCCGGTC TGGTCCAGTG GGCATACAAG 1300

CAGGCTGGTC TGAACCTGCC TCGCACGAGC TACGACCAGG CCGCAGCCTA 1350

AATTT

1355
SEQ ID NO: 3
   SEQUENCE TYPE: Nucleotide
   SEQUENCE LENGTH: 2806 base pairs
   STRANDEDNESS: Single
   TOPOLOGY: Linear
   PROPERTIES: operon 
AGATGCCACT GCGCCATTCC TGGTGAGGTA CGTCGGCCGA CCACCGTTCG 50

GCTGGCGTAC CACATCCACT TTCAAGTCCT GCACATCCGC ACCTTTTTTA 100

AATCGCCAAC TATCGCTATC ATCGTTATAT GACGATAAAT AAAGAGTCGG 150

GGTGCCTCGC CGCAACCGGC AGCGGGTCCA GCTTGGATGC GGCAGTGGCC 200

CTGTTCCACA GTCTCTCGGA CGGCACCCGA TTGTCGATCG TGCGCCGTCT 250

CGCCGAAGGA GAGGCACGGG TTGCGGATTT GATCGGCGAA CTCGGCCTCG 300

CCCAGTCGAC GGTGTCCGCA CATGTCGCAT GCCTACGTGA TTGCGGTTTG 350

GTCGACGGCA GACCGGAAGG TCGGCAGGTG TTCTACTCGC TGGCCCGGCC 400

GGAATTGATG GACCTACTCG CCTCGGCGGA GACGCTCCTT GCCGCGACAG 450

GGAACGCGGT TGCCCTGTGC CCGAATTACG GCACCGACAT CGGAGATAGC 500

CGTGAGTGAC GCGTGCGGCT GCGGCCACGA CGAACCCCGT GCCGAGGGCG 550

AGGAAGAACA CGGGCCCGAA AAGTGGTGGC AGGTTACCGA GATCCGGGCG 600

GCTGCAGCTG CGGGCGTGCT GCTGATCGCG GCCCTGACAG TCGGGCTGGC 650

CGGCGGACCT GATGCGCTCG GGATCGGCCT CGAAGCGGGC GCGCTGATCA 700

TTGCCGGCTA CACCTTCGTA CCGTCCACCC TCACACGCCT GGCCAAGGGC 750

AAAATCGGGG TCGGCACCCT GATGACGATC GCGGCCGTCG GCGCGGTACT 800

GCTCGGCGAG GTCGGCGAAG CAGCCATGCT CGCATTCTTG TTTGCGATCA 850

GCGAGGGACT CGAGGAGTAC GCGGTCACCC GCACCCGCCG TGGCCTGCGC 900

GCGTTACTGT CCCTGGTCCC GGACACCGCG ACAGTGCTCC GCGACGGCCG 950

GGAGGAAACC GTCCCACCCT CGGACCTCGA GCTCGGTGAG GCCATGATCG 1000

TCAAGCCCGG GGAGCGGATC GCCACCGACG GTGTCATTCG CGCCGGCCGC 1050

ACCGCCCTGG ACACCTCCGC GATCACCGGA GAATCGGTGC CCGTCGAAGC 1100

CGGTCCCGGC GACGAGGTAT TCGCCGGGTC GATCAACGGC ACCGGCGTCC 1150

TCGAAGTCGA GGTCACCGCA GAGGCGCAGG ACAATTCGCT GGCGAAGATC 1200
 
GTGCGGATCG TCGAAGCTGA GCAGTCCCGC AAGGGCGAGG CGCAACGCCT 1250

CGCCGACAAG ATCGCCAAAC CGCTGGTTCC GGGCGTAATG ATCGTCGCCG 1300

CGGTTATCGC CGCGGTCGGC AGCGTGCTCG GTGATCCGCT GGTGTGGATC 1350

GAACGAGCCC TGGTGGTACT GGTCGCCGCG TCCCCGTGCG CGCTGGCCAT 1400

CGCGATACCC GTCACGGTCG TCGCCGCAAT CGGCGCAGCA TCCAAGCTCG 1450

GTGTCCTGGT CAAGGGCGGC GCAGCCCTCG AAGCGCTCGG CAGGATCCGC 1500

ACCATCGCAT TGGACAAGAC CGGCACCCTC ACCCGCAACC AACCCGCCGT 1550

GATCGACGTC GCGACCGCGA ACGGCGCCAA CCGCGGTGAC GTTCTCGCGG 1600

TGGCCGCAGC GTTGGAGGCA CGCAGCGAGC ACCCGCTCGC TCGCGCGATT 1650

CTCGCCGCCG TCGAGGACTA CACACCCGCT GATGACGCGG ACGCAGTCAT 1700

CGGTGCAGGC CTGACCGGCT ACATCGACGG CATCCCCGTC CGGCTCGGTC 1750

GACCAGGCTG GATCGACCCG GGGCCACTGA CCGGGGATAT CGAACGAATG 1800

CAGCACGCCG GCGCCACTGC GGTCCTGATC GAACGCGCCG GAACCGTGAT 1850

CGGGGCGGTC GCCGTCCGCG ACGAACTCCG CCCGGAAGCA CGCGAAGTGG 1900

TCACCGAACT ACGCCGCGGC GGCTACACCG TCGCGATGCT CACCGGCGAC 1950

AACGAGCGCA CTGCCCGCGC TCTGGCCGCC GATGTCGGCA TCGACGACGT 2000

GCACGCCGAT CTGCGCCCGG AGGACAAGGC GCGCATTGTG GAGACCTTGC 2050

GTGCGAGCCG ACCGACGGCG ATGGTCGGGG ACGGTGTCAA CGATGCCCCC 2100

GCCCTGGCCA CCGCCGATCT GGGTATCGCG ATGGGTGCGA TGGGAACCGA 2150

CGTCGCCATC GAGACCGCCG ATGTCGCTTT GATGGGCGAG GACCTGCGGC 2200

ATCTTCCTCA GGCTCTGCAG CACGCGCGAC GATCGCGGTC GATCATGTTG 2250

CAGAACGTCG GCCTGTCTTT GGCGATCATC ACCGTTCTGA TGCCTCTGGC 2300

TCTGTTCGGT GTACTCGGAT TGGCTGCGGT CGTGCTCGTC CACGAGGTCG 2350

CCGAGATCGT CGTCATCGCC AACGGGGTGC GCGCCGGGCG TACCCGAAGT 2400

CTGGCTGCTG TACCGCAGTC GACCAGGGCG TCCGAGCCGG CCACGGTAGT 2450
 
ACGGCGATGA CATCTGCCGC CAGCTCCGGG TCATCATCGG TGTCCGTGGT 2500

CGGAATGCGT CGCGGCCGGG GTGTTCTCGT CATACTCGCA GGTGCCTTGG 2550

CTGCGGTCGC GCTGCTCCTC GACCCGGTGG CACGTGGCGC GCTGTCCGGT 2600

GGGCAGGTAG TCGATTTCGG GGTCTTGCAA CTGCGATTGG CCTACAACAG 2650

CGGTGTCGCG TTCAGTCTCG GTGACCAGCT CCCCACAGTC GTCGTTCTCG 2700

CCGGCACCGC TGCCCTCACC GCCGCGATCG GTGTGTTCGC CTGGCGCACA 2750

GCATCTGAGC GTCCCGTACT CCAGACCATC GGGCTGGCCG CGATCACGGC 2800

CGGTACC 2806
SEQ ID NO: 4
   SEQUENCE TYPE: Nucleotide
   SEQUENCE LENGTH: 22 base pairs
   STRANDEDNESS: Single
   TOPOLOGY: Linear
   PROPERTIES: Primer
   CAGTCACGAC GTTGTAAAAC GA
SEQ ID NO: 5
   SEQUENCE TYPE: Nucleotide
   SEQUENCE LENGTH: 20 base pairs
   STRANDEDNESS: Single
   TOPOLOGY: Linear
   PROPERTIES: Primer
   TGCATTTGTC GTTGTTGAGT
SEQ ID NO: 6
   SEQUENCE TYPE: Nucleotide
   SEQUENCE LENGTH: 23 base pairs
   STRANDEDNESS: Single
   TOPOLOGY: Linear
   PROPERTIES: Primer
   GATCGGCCGGG ATCCACGAGT GTT
SEQ ID NO: 7
   SEQUENCE TYPE: Nucleotide
   SEQUENCE LENGTH: 58 base pairs
   STRANDEDNESS: Single
   TOPOLOGY: Linear
   PROPERTIES: Primer 
ACCCAACACT GAGCTGTTAA CGGCCGGAGC GGCCGATGCA

TTTTAGGTGA TGCCCGGG
SEQ ID NO: 8
   SEQUENCE TYPE: Nucleotide
   SEQUENCE LENGTH: 23 base pairs
   STRANDEDNESS: Single
   TOPOLOGY: Linear
   PROPERTIES: Primer
   TGGCCCGGCC GGAATTGATG GAC
SEQ ID NO: 9
   SEQUENCE TYPE: Nucleotide
   SEQUENCE LENGTH: 24 base pairs
   STRANDEDNESS: Single
   TOPOLOGY: Linear
   PROPERTIES: Primer
   GCCGACGGCC GCGATCGTGA TCAG
SEQ ID NO: 10
   SEQUENCE TYPE: Nucleotide
   SEQUENCE LENGTH: 23 base pairs
   STRANDEDNESS: Single
   TOPOLOGY: Linear
   PROPERTIES: Primer
   ATTCGAGAGT GCATATGCGG AAC
SEQ ID NO: 11
   SEQUENCE TYPE: Nucleotide
   SEQUENCE LENGTH: 23 base pairs STRANDEDNESS: Single
   TOPOLOGY: Linear
   PROPERTIES: Primer
   CCATTTCTTC CAAGCTTCCG CCG

## Claims

1. An expression vector which comprises:
(a) the rep genes, ORF81 and trbA which encode proteins involved in replication in Rhodococcus;
(b) the gene parA having the sequence SEQ. ID. No. 1;
(c) a constitutive promoter of Rhodococcus having the sequence SEQ. ID. No. 2;
(d) a multiple cloning site downstream of the promoter;
(e) at least one gene which encodes a genetic marker selected from the genes of the cad operon, which confer resistance to cadmium, or the genes which encode resistance to an antibiotic; and
(f) the replication origin in E.coli.

2. The vector according to claim 1, which comprises downstream of the constitutive promoter one or more genes which encode a protein of interest.

3. The expression vector according to claim 2, wherein the proteins are selected from enzymes involved in the selective removal of organic sulfur from fossil fuels or enzymes involved in the production of L-aminoacids, enantiomorphs of chiral compounds and carboxylic acids.

4. The expression vector according to claim 2, which comprises downstream the constitutive promoter the sox operon which encodes SoxA, SoxB and SoxC enzymes.

5. The expression vector according to claim 1, obtained by:
(1) construction of a cloning plasmid vector comprising:
(a) the rep genes, ORF81 and trbA which encode proteins involved in replication in Rhodococcus;
(b) the gene parA having the sequence SEQ. ID. No. 1; and
(c) at least one gene which encodes a genetic marker selected from the genes of the cad operon, which confer resistance to cadmium, or the genes which encode resistance to an antibiotic;
(2) isolation of a constitutive promoter of Rhodococcus having the sequence SEQ ID No:2; and
(3) insertion of said constitutive promoter in the above cloning vector.

6. A microorganism comprising the expression vectors according to claims 1 to 4, wherein said micro-organism is selected from Rhodococcus, Gordona and Nocardia.

7. A strain of Rhodococcus comprising the expression vector according to claims 1 to 4, deposited with the number CBS 102447.

8. A process for the production of homologous or heterologous proteins of interest which comprises cultivating, under suitable conditions, a micro-organism, comprising the expression vector according to claims 1 to 4.

9. The process according to claim 8, wherein the protein is selected from enzymes involved in the selective removal of organic sulfur from fossil fuels and in the production of L-aminoacids, enantiomorphs of chiral compounds and carboxylic acids.

10. The process according to claim 9, wherein the proteins are Sox enzymes and the strain is Rhodococcus SMV114 CBS 102447.

11. A process for the removal of organic sulfur from fossil fuels **characterized in that** it uses a microorganism selected from Rhodococcus, Gordona and Nocardia comprising the expression vector according to anyone of the claims 1 to 4, containing the sox operon downstream the constitutive promoter.

12. The process according to claim 11, wherein the microorganism is Rhodococcus SMV114 CBS 102447.

13. A constitutive promoter of Rhodococcus comprising the sequence SEQ. ID. No. 2.

## Patentansprüche

1. Expressionsvektor, umfassend:
(a) die rep-Gene ORF81 und trbA, die Proteine codieren, die an der Replikation in *Rhodococcus* beteiligt sind;
(b) das Gen parA, das die Sequenz SEQ. ID. Nr. 1 besitzt;
(c) einen konstitutiven Promotor von *Rhodococcus,* der die Sequenz SEQ. ID. Nr. 2 besitzt;
(d) eine multiple Klonierstelle stromabwärts vom Promotor;
(e) mindestens ein Gen, das einen genetischen Marker codiert, der ausgewählt ist aus den Genen des cad-Operons, die eine Resistenz gegen Cadmium verleihen, oder den Genen, die eine Resistenz gegen ein Antibiotikum codieren; und
(f) den Replikationsursprung von *E.coli*

2. Vektor gemäß Anspruch 1, der stromabwärts vom konstitutiven Promotor ein oder mehrere Gene umfasst, die ein Protein von Interesse codieren.

3. Expressionsvektor gemäß Anspruch 2, wobei die Proteine ausgewählt sind aus Enzymen, die an der selektiven Entfernung von organischem Schwefel aus fossilen Brennstoffen beteiligt sind, oder Enzymen, die an der Produktion von L-Aminosäuren, Enantimorphen chiraler Verbindungen und Carbonsäuren beteiligt sind.

4. Expressionsvektor gemäß Anspruch 2, der stromabwärts vom konstitutiven Promotor das sox-Operon umfasst, das die Enzyme SoxA, SoxB und SoxC codiert.

5. Expressionsvektor gemäß Anspruch 1, erhalten durch:
(1) Konstruktion eines Klonierungsplasmidvektors, der umfasst:
(a) die rep-Gene ORF81 und trbA, die Proteine codieren, die an der Replikation in *Rhodococcus* beteiligt sind;
(b) das Gen parA, das die Sequenz SEQ. ID. Nr. 1 besitzt; und
(c) mindestens ein Gen, das einen genetischen Marker codiert, der ausgewählt ist aus den Genen des cad-Operons, die eine Resistenz gegen Cadmium verleihen, oder den Genen, die eine Resistenz gegen ein Antibiotikum codieren;
(2) Isolierung eines konstitutiven Promotors von *Rhodococcus,* der die Sequenz SEQ. ID. Nr. 2 besitzt; und
(3) Insertion dieses konstitutiven Promotors in den obigen Klonierungsvektor.

6. Mikroorganismus, der den Expressionsvektor gemäß Ansprüchen 1 bis 4 umfasst, wobei dieser Mikroorganismus ausgewählt ist aus *Rhodococcus, Gordona* und *Nocardia.*

7. *Rhodococcus-*Stamm, der den Expressionsvektor gemäß Ansprüchen 1 bis 4 umfasst, der unter der Nummer CBS 102447 hinterlegt ist.

8. Verfahren zur Herstellung homologer oder heterologer Proteine von Interesse, das ein Kultivieren eines Mikroorganismus, der den Expressionsvektor gemäß Ansprüchen 1 bis 4 umfasst, unter geeigneten Bedingungen umfasst.

9. Verfahren gemäß Anspruch 8, wobei das Protein ausgewählt ist aus Enzymen, die an der selektiven Entfernung von organischem Schwefel aus fossilen Brennstoffen und an der Produktion von L-Aminosäuren, Enantiomorphen chiraler Verbindungen und Carbonsäuren beteiligt sind.

10. Verfahren gemäß Anspruch 9, wobei die Proteine Sox-Enzyme sind und der Stamm *Rhodococcus* SMV114 CBS 102447 ist.

11. Verfahren zur Entfernung von organischem Schwefel aus fossilen Brennstoffen, **dadurch gekennzeichnet, dass** es einen Mikroorganismus verwendet, der ausgewählt ist aus *Rhodococcus, Gordona* und *Nocardia,* die den Expressionsvektor gemäß einem der Ansprüche 1 bis 4 umfassen, der stromabwärts vom konstitutiven Promotor das sox-Operon enthält.

12. Verfahren gemäß Anspruch 11, wobei der Mikroorganismus *Rhodococcus* SMV114 CBS 102447 ist.

13. Konstitutiver Promotor von *Rhodococcus,* der die Sequenz SEQ. ID. Nr. 2 umfasst.

## Revendications

1. Vecteur d'expression, comprenant :
a) les gènes *rep, ORF81* et *trbA,* qui codent des protéines impliquées dans la réplication chez Rhodococcus ;
b) le gène *parA,* dont la séquence est donnée en tant que Séquence n° 1 ;
c) un promoteur constitutif de Rhodococcus, dont la séquence est donnée en tant que Séquence n° 2 ;
d) un site de clonage multiple, placé en aval du promoteur ;
e) au moins un gène codant un marqueur génétique, choisi parmi les gènes de l'opéron *cad,* qui confèrent une résistance au cadmium, et les gènes qui confèrent une résistance à un antibiotique; et
f) l'origine de réplication de E. coli.

2. Vecteur conforme à la revendication 1, qui comprend, en aval du promoteur constitutif, un ou plusieurs gène(s) codant une protéine intéressante.

3. Vecteur d'espression conforme à la revendication 2, les protéines étant choisies parmi des enzymes impliquées dans l'élimination sélective de composés organiques soufrés de combustibles fossiles et des enzymes impliquées dans la production d'acides aminés L, d'énantiomorphes de composés chiraux, ou d'acides carboxyliques.

4. Vecteur d'expression conforme à la revendication 2, qui comprend, en aval du promoteur constitutif, l'opéron *sox* qui code les enzymes SoxA, SoxB et SoxC.

5. Vecteur d'expression conforme à la revendication 1, obtenu par les opérations suivantes:
1) construction d'un plasmide vecteur de clonage, comprenant:
a) les gènes *rep, ORF81* et *trbA,* qui codent des protéines impliquées dans la réplication chez Rhodococcus,
b) le gène *parA,* dont la séquence est donnée en tant que Séquence n° 1, et
c) au moins un gène codant un marqueur génétique, choisi parmi les gènes de l'opéron *cad,* qui confèrent une résistance au cadmium, et les gènes qui confèrent une résistance à un antibiotique;
2) isolement d'un promoteur constitutif de Rhodococcus, dont la séquence est donnée en tant que Séquence n° 2 ; et
3) insertion de ce promoteur constitutif dans ledit vecteur de clonage.

6. Microorganisme comprenant un vecteur d'expression conforme aux revendications 1 à 4, lequel microorganisme est choisi parmi ceux des genres Rhodococcus, Gordona et Nocardia.

7. Souche de Rhodococcus comprenant un vecteur d'expression conforme aux revendications 1 à 4, déposée, portant le numéro CBS 102447.

8. Procédé de production de protéines intéressantes, homologues ou hétérologues, qui comprend le fait de cultiver, dans des conditions appropriées, un microorganisme comprenant un vecteur d'expression conforme aux revendications 1 à 4.

9. Procédé conforme à la revendication 8, dans lequel la protéine est choisie parmi des enzymes impliquées dans l'élimination sélective de composés organiques soufrés de combustibles fossiles ou dans la production d'acides aminés L, d'énantiomorphes de composés chiraux, ou d'acides carboxyliques.

10. Procédé conforme à la revendication 9, dans lequel les protéines sont les enzymes Sox et la souche est Rhodococcus SMV114 (CBS 102447).

11. Procédé permettant d'éliminer des composés organiques soufrés de combustibles fossiles, **caractérisé en ce qu'**on se sert d'un microorganisme choisi parmi ceux des genres Rhodococcus, Gordona et Nocardia, qui comprend un vecteur d'expression conforme aux revendications 1 à 4, comportant l'opéron *sox* placé en aval du promoteur constitutif.

12. Procédé conforme à la revendication 11, dans lequel le microorganisme est la souche Rhodococcus SMV114 (CBS 102447).

13. Promoteur constitutif de Rhodococcus, comprenant la séquence donnée en tant que Séquence n° 2.
